# EUROPEAN PATENT APPLICATION

(11) **EP 3 628 216 A1**
(43) Date of publication of application: **01.04.2020**
(21) Application number: 19196315.6
(22) Date of filing: 10.09.2019
(51) Int. Cl.: A61B 5/01, A61B 5/00, A61B 5/02

(54) **SENSING ASSEMBLY**

(30) Priority: 25.09.2018 US 201862736418 P; 25.09.2018 US 201862736426 P
(71) Applicant: IWEECARE Co., Ltd., Taipei City 112 (TW)
(72) Inventor: TSENG, Chun-Hao, Taipei City 112 (TW); CHANG, Ho-Yi, New Taipei City 242 (TW)
(74) Representative: Emde, Eric

(57) **Abstract**

A sensing assembly including a sensing device and a binding component is provided. The sensing device includes a battery module, a circuit board module, and a physical condition sensor. The circuit board module covers a top surface, a bottom surface opposite to the top surface, and a side surface connecting the top surface and the bottom surface of the battery module. The physical condition sensor is disposed on an outer surface of the circuit board module and is configured to contact a sensing region of a user to detect a physical condition signal of the sensing region. The binding component is coupled to the sensing device to fix the sensing device to the sensing region of the user.

## Description

### BACKGROUND

### Technical Field

The disclosure relates to a device assembly, and more particularly to a sensing assembly.

### Description of Related Art

Physical condition sensing devices are commonly used throughout the world currently. Such devices normally rely on some form of physical contact to measure the user's temperature or other physical parameters through contact with the user's skin. Monitoring physical condition as auxiliary means to home care and supervision for the elderly has become increasingly necessary. The same also applies to infants, children, women, pregnant women, athletes, etc., who may be unable to participate actively in physical condition monitoring. In view of this, various methods for patient monitoring have been developed so far, which are often carried out in hospitals or together with other healthcare personnel. Although such monitoring equipment are useful, the size of current physical condition sensing devices is still bulky. Therefore, sensing devices which directly contact the user have the shortcoming of causing discomfort to the user.

### SUMMARY

The disclosure provides a sensing assembly, which is small in size and can be easily fixed onto the body surface of the user, thereby improving the user's comfort during use.

In an embodiment of the disclosure, a sensing assembly includes a sensing device and a binding component. The sensing device includes a battery module, a circuit board module, and a physical condition sensor. The circuit board module covers a top surface, a bottom surface opposite to the top surface, and a side surface connecting the top surface and the bottom surface of the battery module. The physical condition sensor is disposed on an outer surface of the circuit board module and is configured to contact a sensing region of a user to detect a physical condition signal of the sensing region. The binding component is coupled to the sensing device to fix the sensing device to the sensing region of the user.

In an embodiment of the disclosure, the circuit board module is a flexible circuit board, which is bent to cover the top surface, the bottom surface, and the side surface.

In an embodiment of the disclosure, the circuit board module includes a first circuit board, a second circuit board, and a connector. The first circuit board covers the top surface and includes a plurality of first pins, the second circuit board covers the bottom surface and includes a plurality of second pins, and the connector covers the side surface and the opposite ends thereof are respectively engaged with the first pins and the second pins to electrically connect the first circuit board and the second circuit board.

In an embodiment of the disclosure, the circuit board module includes a first circuit board and a second circuit board. The first circuit board covers the top surface and includes a plurality of pins, the second circuit board covers the bottom surface and includes a connector corresponding to the pins. The connector covers the side surface and engages with the pins to electrically connect the first circuit board and the second circuit board.

In an embodiment of the disclosure, the circuit board module includes a first circuit board, a flexible circuit board, and a second circuit board. The first circuit board covers the top surface and is connected to the flexible circuit board, the second circuit board covers the bottom surface and includes a connector, and the flexible circuit board is bent to cover the side surface and to engage with the connector to electrically connect the first circuit board and the second circuit board.

In an embodiment of the disclosure, the circuit board module includes a flexible circuit board and a printed circuit board. The flexible circuit board is bent to cover the top surface and the side surface, and the printed circuit board covers the bottom surface and includes a connector engaged with the flexible circuit board to electrically connect the flexible circuit board and the printed circuit board.

In an embodiment of the disclosure, the sensing assembly further includes a printed antenna printed on the circuit board module.

In an embodiment of the disclosure, the sensing assembly further includes a wireless communication switch disposed on the circuit board module and electrically connected to the printed antenna.

In an embodiment of the disclosure, the sensing assembly further includes an isolation layer encapsulating the battery module and the circuit board module, and exposing at least a portion of the physical condition sensor.

In an embodiment of the disclosure, the material of the isolation layer includes a thermoplastic material and the isolation layer may be formed through injection molding.

In an embodiment of the disclosure, the sensing region is a groin of the user.

In an embodiment of the disclosure, the binding component includes a close-fitting bottom which has an inner pocket to accommodate the sensing device.

In an embodiment of the disclosure, the binding component includes an elastic band to be fastened to a groin of the user.

In an embodiment of the disclosure, the binding component includes an adhesive layer to be disposed on a portion of the circuit board module covering the bottom surface.

In an embodiment of the disclosure, the binding component includes an adhesive layer to be disposed on a portion of the circuit board module covering the top surface and a peripheral region of the adhesive layer is attached to the user.

In an embodiment of the disclosure, the sensing device includes a plurality of sensing devices and the plurality of sensing devices are configured to wirelessly communicate with a reading device.

In an embodiment of the disclosure, the sensing region is under an armpit of the user.

In an embodiment of the disclosure, the binding component includes a double-sided tape having one side disposed on the circuit board module and the other side being attached to the user.

To make the aforementioned and other features of the disclosure more comprehensible, several embodiments accompanied with drawings are described in detail as follows.

### BRIEF DESCRIPTION OF THE DRAWINGS

FIG. 1A is a cross-sectional schematic view of a sensing assembly in accordance with an embodiment of the disclosure.
FIG. 1B is a schematic view illustrating the sensing assembly of FIG. 1A being attached to an armpit of a user in accordance with an embodiment of the disclosure.
FIG. 2 is a top schematic view of a sensing assembly in accordance with an embodiment of the disclosure.
FIG. 3 is a bottom schematic view of a sensing assembly in accordance with an embodiment of the disclosure.
FIG. 4 is a bottom schematic view of a sensing device in accordance with an embodiment of the disclosure.
FIG. 5 is a cross-sectional schematic view of a portion of a sensing device in accordance with an embodiment of the disclosure.
FIG. 6 is a cross-sectional schematic view of a portion of a sensing device in accordance with an embodiment of the disclosure.
FIG. 7 is a cross-sectional schematic view of a portion of a sensing device in accordance with an embodiment of the disclosure.
FIG. 8 is cross-sectional schematic view of a portion of a sensing device in accordance with an embodiment of the disclosure.
FIG. 9 is a schematic view of a sensing assembly in accordance with an embodiment of the disclosure.
FIG. 10 is a schematic view of a sensing assembly in accordance with an embodiment of the disclosure.
FIG. 11 is a block diagram of a sensing assembly in accordance with an embodiment of the disclosure.

### DESCRIPTION OF THE EMBODIMENTS

The aforementioned and other technical content, characteristics, and effects regarding the disclosure shall be clearly presented in conjunction with detailed illustrations of various embodiments with reference to the drawings. The directional terms, for example, "up", "down", "front", "back", "left", "right", etc., mentioned in the following embodiments are only directions with reference to the drawings. Therefore, the directional terms used are for the purpose of illustration and are not to limit the disclosure. In addition, in the following embodiments, the same or similar components are denoted by the same or similar reference numerals.

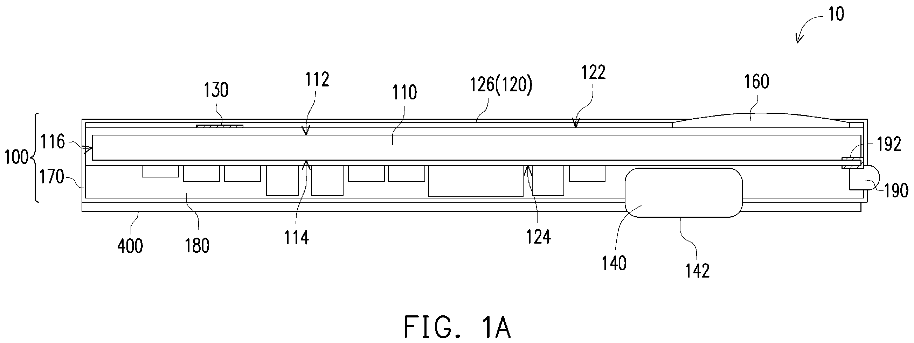

FIG. 1A is a cross-sectional schematic view of a sensing assembly in accordance with an embodiment of the disclosure. Referring to FIG. 1A, in the present embodiment, a sensing assembly 10 may include a sensing device 100 and a binding component 400, wherein the sensing device 100 may be fixed to a specific part of the user's body via the binding component 400 and is configured to detect the physical condition of the user. For example, the sensing assembly 10 may be fixed to parts such as the chest, wrist, armpit, forehead, front of nostrils, back, groin, etc. to detect physical parameters such as the pulse, heart rate, body temperature, perspiration, breathing, skin moisture, etc. of the user. Of course, the disclosure is not limited thereto.

In general, a basal body temperature (abbreviated as BBT or BTP) is the lowest body temperature reached during rest (usually during sleep). The BBT is usually measured at a time right after waking up and before carrying out any activity, but a value slightly higher than the actual BBT may still be obtained. Therefore, in the embodiment, the sensing assembly 10 is configured to be fixed onto the body surface of the user so that the sensing assembly 10 may (continuously) monitor and detect the BBT of the user during sleep. Thus, the BBT detected by the sensing assembly 10 of the disclosure can better reflect the actual BBT.

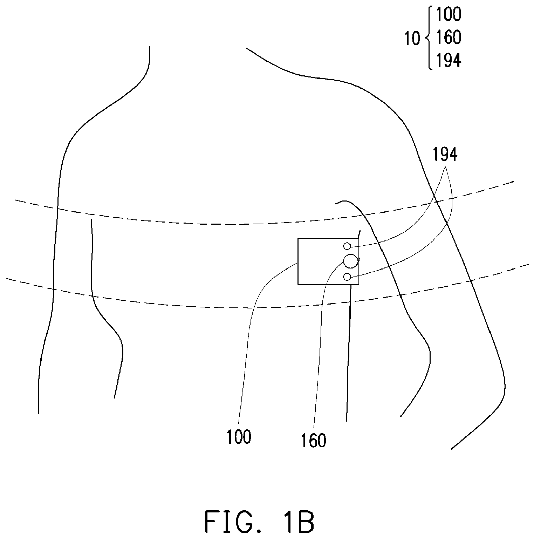

FIG. 1B is a schematic view illustrating the sensing assembly 10 of FIG. 1A being attached to an armpit of the user in accordance with an embodiment of the disclosure. The binding component 400 is coupled to the sensing device 100 to fix the sensing device 100 to a sensing region (for example, parts such as an armpit, a groin, etc.) of the user. In the embodiment, the sensing device 100 may be attached to the armpit region (at above the chest line) of the user via the binding component 400, such as a double-sided tape, etc., so as to sense detect the physical parameters such as the pulse, heart rate, body temperature, perspiration, breathing, skin moisture, etc. of the user. The binding component 400 may have one side being attached to the sensing device 100 and the other side adhered to the armpit region of the user. In general, the armpit region refers to a region extending from the underside of the shoulder joint (also called as axilla) to above the chest line of a human body (as shown in FIG. 1B). The most common way to take a body temperature (i.e., an axillary method) is under the armpit (namely, at the armpit region), and thus, in the embodiment, the sensing device 100 is configured to perform measurements under the armpit of the user.

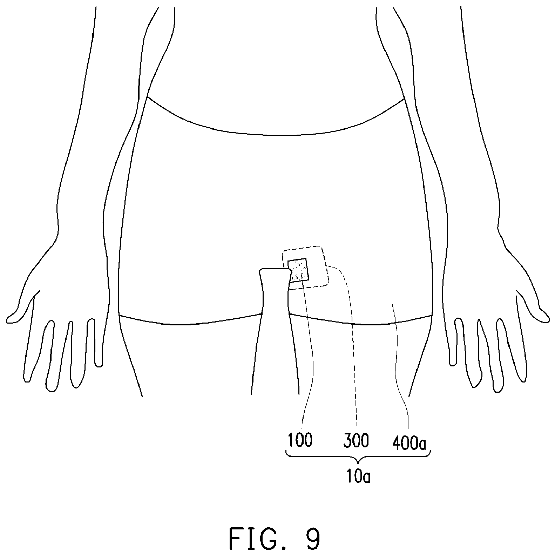

Moreover, many experimental data have demonstrated that the temperature at the groin can better reflects the actual BBT and is less sensitive to the effect of heat generated by brown adipose tissue. Therefore, in other embodiments, the sensing device 100 may be configured to be fixed to the groin of the user through the binding component 400, such as a double-sided tape, etc. In general, the groin refers to the groove at the junction connecting the abdomen and the legs of a human body. The regions nearby may all be called the groin, which is mostly located on both sides of genitalia around the inner thigh area (as shown in FIG. 9).

In some embodiments, the sensing device 100 may include a battery module 110, a circuit board module 120, and a physical condition sensor 140. In the embodiment, the binding component 400 may be an adhesive layer disposed on the circuit board module 120 to attach the sensing device 100 to the sensing region of the user. The circuit board module 120 covers a top surface 112, a bottom surface 114 opposite to the top surface 112, and a side surface 116 connecting the top surface 112 and the bottom surface 114 of the battery module 110. In the embodiment, the battery module 110 may be lithium battery, button battery, etc., but the embodiment is not limited thereto. The battery module 110 may be rectangular and has a plurality of side surfaces 116. The circuit board module 120 may cover one of the plurality of side surfaces 116 of the battery module 110. In other embodiments, the battery module 110 may also be cylindrical. The embodiment does not limit the form of the battery module 110. The physical condition sensor 140 is disposed on an outer surface of the circuit board module 120 and is configured to contact the sensing region of the user to detect a physical condition signal of the sensing region. In the embodiment, the sensing device 100 further includes a printed antenna 130 printed on the circuit board module 120.

Specifically, the circuit board module 120 covers the top surface 112, the bottom surface 114, and the side surface 116 of the battery module 110 as shown in FIG. 1A and is electrically connected to the battery module 110. In the embodiment, the circuit board module 120 may be a flexible printed circuit (FPC) board 126 and the FPC board 126 is bent to cover the top surface 112, the bottom surface 114, and the side surface 116 of the battery module 110. Therefore, the FPC board 126 can be bent owing to its flexibility to electrically connect the two opposite surfaces, i.e. the top surface 112 and the bottom surface 114 of the battery module 110, such that the components of the sensing device 100 may be disposed on the two opposite surfaces of the battery module 110, so as to increase the space utilization of the sensing device 100, thereby reducing the overall size of the sensing device 100. In addition, the FPC board 126 provides softness and flexibility to the sensing device 100, thereby providing better comfort to the user when the sensing assembly 10 is in direct contact with the sensing region of the user.

In particular, the length of the sensing device 100 may be minimized to approximately 2.6 cm and the width of the sensing device 100 may be minimized to approximately 2 cm. As for the thickness, the circuit board module 120 and the battery module 110 together form the core portion of the sensing device 100, and the thickness of the core portion may be minimized to approximately 0.92 mm. Of course, the numerical ranges listed herein are for illustrative purposes only and the disclosure is not limited herein.

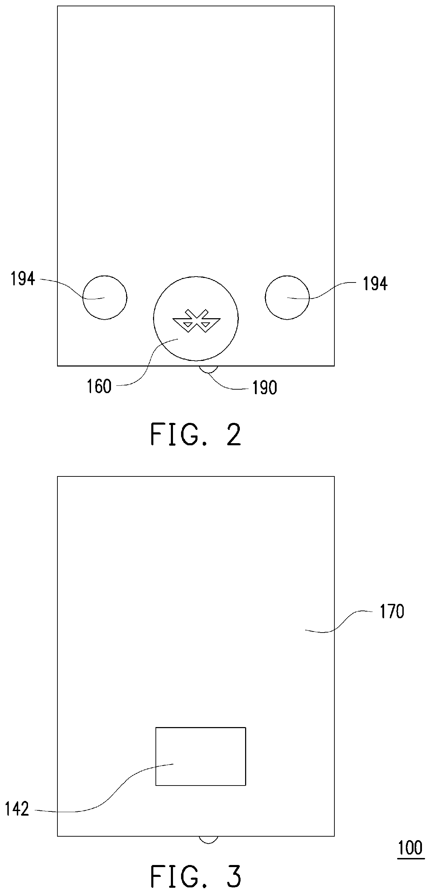

FIG. 2 is a top schematic view of a sensing assembly in accordance with an embodiment of the disclosure. FIG. 3 is a bottom schematic view of a sensing assembly in accordance with an embodiment of the disclosure. Referring to FIGs. 1 to 3, in the embodiment, the circuit board module 120 may include a first outer surface 122 and a second outer surface 124 opposite to the first outer surface 122. The printed antenna 130 may be formed on the first outer surface 122 via, for example, a printing process. The physical condition sensor 140 is disposed on the second outer surface 124 of the circuit board module 120. In other words, the printed antenna 130 and the physical condition sensor 140 are respectively disposed on the two opposite surfaces of the circuit board module 120 to maintain a certain distance between the printed antenna 130 and the physical condition sensor 140, so as to prevent interference between the two without increasing the overall length of the sensing device 100.

In some embodiments, the physical condition sensor 140 includes a sensor region 142 for contacting the user to detect the physical condition parameter of the user. The material of the sensor region 142 may be metal, for example, stainless steel, nickel, copper, gold, silver, etc. Of course, embodiments of the disclosure are not limited thereto. In the embodiment, the physical condition sensor 140 may be a thermometer for measuring the body temperature of the user. Of course, the embodiment is for illustrative purposes only and is not intended to limit the embodiments of the disclosure. The sensing device 100 may further include a wireless communication switch 160 as shown in FIG. 1A and FIG. 2. The wireless communication switch 160 is the switch for the wireless communication module. The wireless communication switch 160 is disposed on the first outer surface 122, and is electrically connected to the printed antenna 130, so that the physical condition signal detected by the physical condition sensor 140 may be uploaded to the cloud via, for example, Bluetooth, Wi-Fi, 4G, 5G, Long Term Evolution (LTE), etc. and by pressing the wireless communication switch 160 to turn on wireless communication. In the embodiment, the sensing device 100 may use the internet (online) and the telecommunication network (offline) to perform real time alert and/or physical condition prediction (for example, for labor) to notify the caregiver in time.

In addition, the circuit board module 120 further includes a power source switch 190, such as a power source pad 192 as shown in FIG. 1A and a charging pad 194 as shown in FIG. 2. The power source pad 192 is in contact with the battery module 110 to electrically connect the battery module 110 and the circuit board module 120. Therefore, the user may turn on and turn off the sensing device 100 by pressing the power source switch 190. The charging pad 194 may be disposed on the first outer surface 122 and electrical connects to the battery module 110. The charging pad 194 as shown in FIG. 2 is exposed so that the charging pad 194 may be connected to a charger to charge the battery module 110 using a wall charger or a USB port.

In some embodiments, the sensing device 100 may further include an isolation layer 170. The isolation layer 170 encapsulates the battery module 110 and the circuit board module 120 to prevent water from seeping into the sensing device 100. In the embodiment, the isolation layer 170 may further encapsulate other components, parts, wafer sets of the sensing device 100, etc. The isolation layer 170 exposes at least a portion, such as the sensor region 142, of the physical condition sensor 140, the charging pad 194, and the wireless communication switch 190 as shown in FIG. 2 and FIG. 3. In the embodiment, the isolation layer 170 may be formed by injection molding. Therefore, the material of the isolation layer 170 may include a thermoplastic material such as polyethylene terephthalate (PET), silicone, thermoplastic polyurethane (TPU), thermoplastic rubber (TPR), thermoplastic elastomer (TPE), thermoplastic silicone vulcanizate (TPSiV®), or other suitable materials. In addition, the sensing device 100 may further include an isolation coating 180 filled between the isolation layer 170 and the circuit board module 120. The isolation coating 180 may include an electrically insulating coating and a waterproof coating.

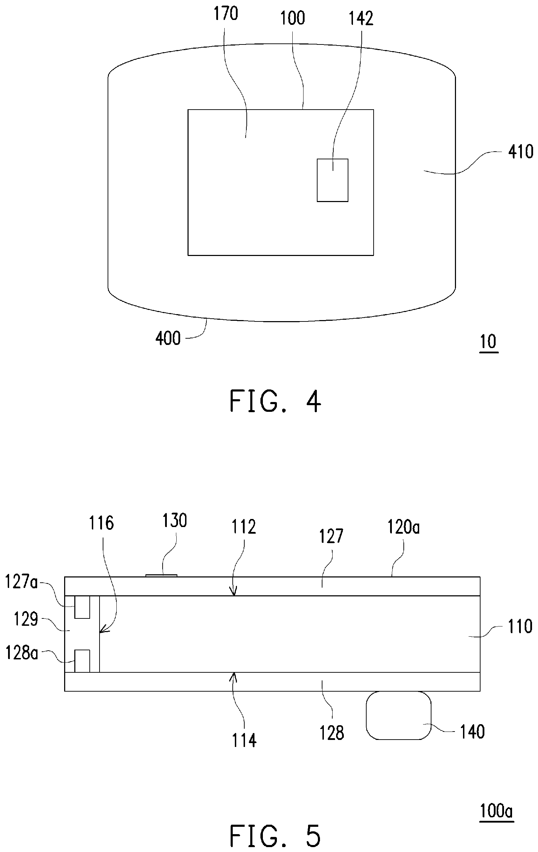

FIG. 4 is a bottom schematic view of a sensing device in accordance with an embodiment of the disclosure. Referring to FIG. 1A and FIG. 4, in the embodiment shown in FIG. 1A, the binding component 400 may be an adhesive layer such as a double-sided tape, which is disposed on the second outer surface 124 of the circuit board module 120. More specifically, the binding component 400 is disposed on a portion of the isolation layer 170 corresponding to the second outer surface 124, such that the sensing device 100 may be attached to the body (i.e. the sensing region) of the user through the binding component 400. The binding component 400 exposes the sensor region 142 of the physical condition sensor 140 such that when the sensing device 100 is attached to the body of the user, the sensor region 142 may be in contact with the body (i.e. the sensing region) of the user. However, in the embodiment shown in FIG. 4, the binding component 400 may also be a single-sided tape disposed on the first outer surface 122. The size of the binding component 400 is substantially larger than the size of the core portion (i.e. the battery module 110 and the circuit board module 120) of the sensing device 100. Therefore, when the binding component 400 is disposed on the first outer surface 122, a peripheral region 410 of the binding component 400 may be exposed, such that the sensing device 100 may be attached to the body of the user via the peripheral region 410 of the binding component 400.

FIG. 5 is a cross-sectional schematic view of a portion of a sensing device in accordance with an embodiment of the disclosure. It should be noted that a sensing device 100a of the present embodiment is similar to the sensing device 100 shown in FIG. 1A. Therefore, the present embodiment continues to use the reference numerals and some content of the foregoing embodiment, wherein the same reference numerals are used to denote the same or similar components and descriptions of the same technical content are omitted. For the descriptions of the omitted part, references may be made to the foregoing embodiment, which will not be reiterated in the present embodiment. Moreover, the sensing device 100a as shown in FIG. 5 only shows the core portion (i.e. the circuit board module and the battery module) thereof, and detailed descriptions of other features similar to or the same as the foregoing embodiment are omitted herein. Referring to FIG. 5, differences between the sensing device 100a of the present embodiment and the sensing device 100 shown in FIG. 1A will be illustrated below.

In the present embodiment, the circuit board module 120a includes a first circuit board 127, a second circuit board 128, and a connector 129. The first circuit board 127 covers the top surface 112 of the battery module 110 and the printed antenna 130 is disposed on the first circuit board 127. The second circuit board 128 covers the bottom surface 114 of the battery module 110 and the physical condition sensor 140 is disposed on the bottom surface 114 of the battery module 110. The connector 129 covers the side surface 116 and connects the first circuit board 127 and the second circuit board 128 to electrically connect the first circuit board 127 and the second circuit board 128. More specifically, the first circuit board 127 further includes a plurality of first pins 127a. The second circuit board 128 further includes a plurality of second pins 128a. The opposite ends of the connector 129 are respectively engaged with the first pins 127a and the second pins 128a to electrically connect the first circuit board 127 and the second circuit board 128.

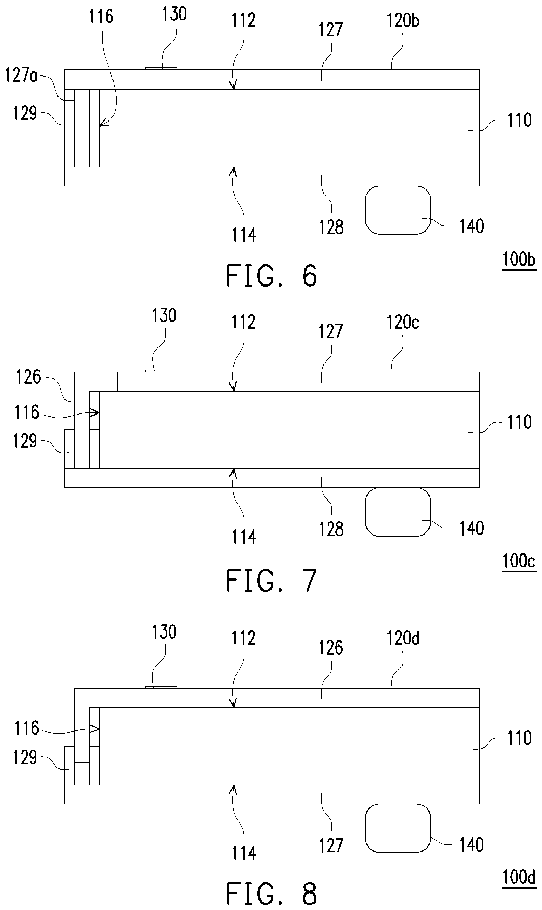

FIG. 6 is a cross-sectional schematic view of a portion of a sensing device in accordance with an embodiment of the disclosure. It should be noted that a sensing device 100b of the present embodiment is similar to the sensing device 100 shown in FIG. 1A. Therefore, the present embodiment continues to use the reference numerals and some content of the foregoing embodiments, wherein the same reference numerals are used to denote the same or similar components and descriptions of the same technical content are omitted. For the descriptions of the omitted part, references may be made to the foregoing embodiments, which will not be reiterated in the present embodiment. Moreover, the sensing device 100b as shown in FIG. 6 only shows the core portion (i.e. the circuit board module and the battery module) thereof, and detailed descriptions of other features similar to or the same as the foregoing embodiments are omitted herein. Referring to FIG. 6, differences between the sensing device 100b of the present embodiment and the sensing device 100 shown in FIG. 1A will be illustrated below.

In the present embodiment, the circuit board module 120b includes a first circuit board 127 and a second circuit board 128. The first circuit board 127 covers the top surface 112 of the battery module 110 and includes a plurality of pins 127a. The printed antenna 130 is disposed on the first circuit board 127. The second circuit board 128 covers the bottom surface 114 of the battery module 110 and includes a connector 129 corresponding to the pins 127a. The physical condition sensor 140 is disposed on the second circuit board 128. The connector 129 covers the side surface 116 and engages with the pins 127a to electrically connect the first circuit board 127 and the second circuit board 128.

FIG. 7 is a cross-sectional schematic view of a portion of a sensing device in accordance with an embodiment of the disclosure. It should be noted that a sensing device 100c of the present embodiment is similar to the sensing device 100 shown in FIG. 1A. Therefore, the present embodiment continues to use the reference numerals and some content of the foregoing embodiments, wherein the same reference numerals are used to denote the same or similar components and descriptions of the same technical content are omitted. For the descriptions of the omitted part, references may be made to the foregoing embodiments, which will not be reiterated in the present embodiment. Moreover, the sensing device 100c as shown in FIG. 7 only shows the core portion (i.e. the circuit board module and the battery module) thereof, and detailed descriptions of other features similar to or the same as the foregoing embodiments are omitted herein. Referring to FIG. 7, differences between the sensing device 100c of the present embodiment and the sensing device 100 shown in FIG. 1A will be illustrated below.

In the present embodiment, the circuit board module 120c includes a first circuit board 127, an FPC board 126, and a second circuit board 128. The first circuit board 127 covers the top surface 112 of the battery module 110 and is connected to the FPC board 126. The printed antenna 130 is disposed on the first circuit board 127. The second circuit board 128 covers the bottom surface 114 of the battery module 110 and includes the connector 129. The physical condition sensor 140 is disposed on the second circuit board 128. The FPC board 126 is bent to cover the side surface 116 and to be engaged with the connector 129 to electrically connect the first circuit board 127 and the second circuit board 128.

FIG. 8 is cross-sectional schematic view of a portion of a sensing device in accordance with an embodiment of the disclosure. It should be noted that a sensing device 100d of the present embodiment is similar to the sensing device 100 shown in FIG. 1A. Therefore, the present embodiment continues to use the reference numerals and some content of the foregoing embodiments, wherein the same reference numerals are used to denote the same or similar components and descriptions of the same technical content are omitted. For the descriptions of the omitted part, references may be made to the foregoing embodiments, which will not be reiterated in the present embodiment. Moreover, the sensing device 100d as shown in FIG. 8 only shows the core portion (i.e. the circuit board module and the battery module) thereof, and detailed descriptions of other features similar to or the same as the foregoing embodiments are omitted herein. Referring to FIG. 8, differences between the sensing device 100d of the present embodiment and the sensing device 100 shown in FIG. 1A will be illustrated below.

In the present embodiment, the circuit board module 120d includes an FPC board 126 and a printed circuit board 127. The FPC board 126 is configured to be bent to cover the top surface 112 and the side surface 116 of the battery module 110. The printed antenna 130 is disposed on the FPC board 126. The printed circuit board 127 covers the bottom surface 114 of the battery module 110 and includes the connector 129. The physical condition sensor 140 is disposed on the printed circuit board 127. The FPC board 126 is bent to cover the side surface 116 and to be engaged with the connector 129 to electrically connect the printed circuit board 127.

FIG. 9 is a schematic view of a sensing assembly in accordance with an embodiment of the disclosure. It should be noted that a sensing assembly 10a of the present embodiment is similar to the sensing assembly 10 shown in FIG. 1A. Therefore, the present embodiment continues to use the reference numerals and some content of the foregoing embodiments, wherein the same reference numerals are used to denote the same or similar components and descriptions of the same technical content are omitted. For the descriptions of the omitted part, references may be made to the foregoing embodiments, which will not be reiterated in the present embodiment. Moreover, detailed descriptions of features of the sensing device 100 of the sensing assembly 10a as shown in FIG. 9 similar to or the same as the foregoing embodiments are omitted herein. Referring to FIG. 9, differences between the sensing assembly 10a of the present embodiment and the sensing assembly 10 shown in FIG. 1A will be illustrated below.

Referring to FIG. 9, in some embodiments, the sensing assembly 10a may be an accessory worn by the user. For example, a binding component 400a of the sensing assembly 10a may be a close-fitting bottom, which may include an inner pocket 300. The close-fitting bottom 400a may be briefs, underwear, shorts, underpants, etc. The inner pocket 300 may be stitched to the inner surface of the close-fitting bottom 400a and is configured to be in contact with the skin (for example, the skin at the groin) of the user. When the user puts on the close-fitting bottom 400a, the position of the inner pocket 300 may correspond to the groin region of the user. The inner pocket 300 may be used to accommodate the sensing device 100. In the present embodiment, the sensing device 100 herein may have the same or at least a similar structure as the sensing device 100 shown in FIG. 1A. In some embodiments, the inner pocket 300 may have opening for exposing the sensor region (for example, the sensor region 142 shown in FIG. 1A) of the physical condition sensor (for example, the physical condition sensor 140 shown in FIG. 1A). In some embodiments, the sensing device 100 may be disposed in the inner pocket 300 or fixed to the inner surface of the close-fitting bottom 400a using other methods. For example, the sensing device 100 may be directly attached to the close-fitting bottom 400a instead of being disposed in the inner pocket 300. Therefore, when the user puts on the close-fitting bottom 400a, the sensing device 100 may detect and track the temperature of the user's groin region.

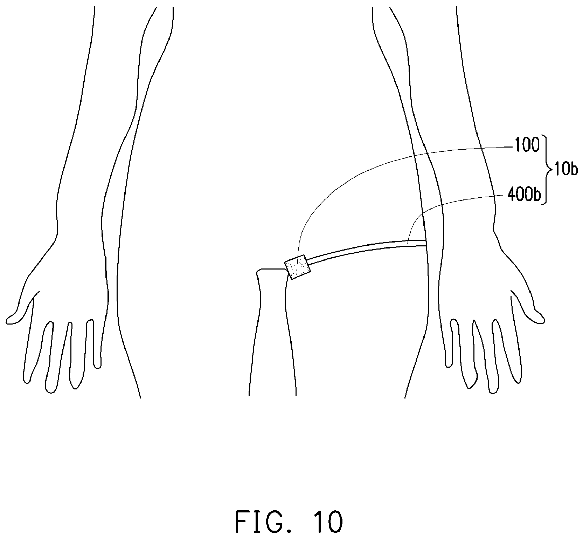

FIG. 10 is a schematic view of a sensing assembly in accordance with an embodiment of the disclosure. It should be noted that a sensing assembly 10b of the present embodiment is similar to the sensing assembly 10 shown in FIG. 1A. Therefore, the present embodiment continues to use the reference numerals and some content of the foregoing embodiments, wherein the same reference numerals are used to denote the same or similar components and descriptions of the same technical content are omitted. For the descriptions of the omitted part, references may be made to the foregoing embodiments, which will not be reiterated in the present embodiment. Moreover, detailed descriptions of features of the sensing device 100 of the sensing assembly 10b as shown in FIG. 10 similar to or the same as the foregoing embodiments are omitted herein. Referring to FIG. 10, differences between the sensing assembly 10b of the present embodiment and the sensing assembly 10 shown in FIG. 1A will be illustrated below.

In the present embodiment, referring to FIG. 10, the sensing device 100 may be fixed onto the binding component 400b and the binding component 400b may be an elastic band fastened to the groin region of the user. Therefore, when the binding component 400b is tied to the groin region of the user, the sensing device 100 is in contact with the groin region of the user to sense the BBT of the groin region. In some embodiments, the binding component 400b may include a joint piece, such as a buckle, to fix the binding component 400b to the groin region (for example, the highest point of the thigh) of the user. In other embodiments, the binding component 400b may be an integrally formed elastic band, which is sleeved around the groin region of the user. Other methods of fixing the sensing device 100 to the groin region of the user to detect and track the BBT of the groin region are also applicable to the embodiments of the disclosure.

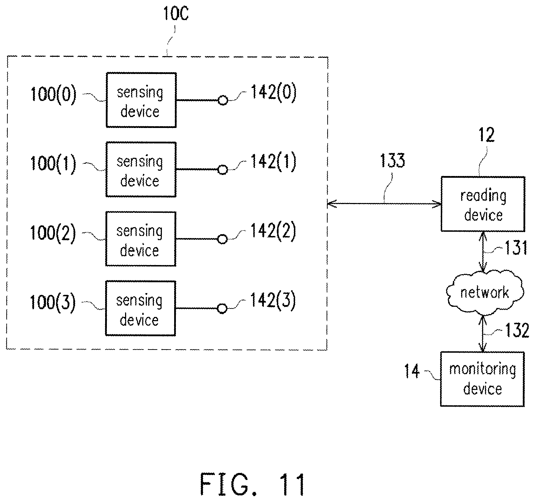

FIG. 11 is a block diagram of a sensing assembly in accordance with an embodiment of the disclosure. It should be noted that the sensing assembly 10c of the present embodiment is similar to the sensing assembly 10 shown in FIG. 1A. Therefore, the present embodiment continues to use the reference numerals and some content of the foregoing embodiments, wherein the same reference numerals are used to denote the same or similar components and descriptions of the same technical content are omitted. For the descriptions of the omitted part, references may be made to the foregoing embodiments, which will not be reiterated in the present embodiment. Referring to FIG. 11, differences between the sensing assembly 10c of the present embodiment and the sensing assembly 10 shown in FIG. 1A will be illustrated below.

Referring to FIG. 11, in the embodiment, the number of sensing devices 100(0), 100(1), 100(2), and 100(3) of the sensing assembly 10c is multiple, and the sensing devices 100(0), 100(1), 100(2), and 100(3) are configured to be in wireless communication with a reading device 12. For example, the sensing assembly 10c includes the plurality of sensing devices 100(0), 100(1), 100(2), and 100(3), and a plurality of sensing electrodes 142(0), 142(1), 142(2), 142(3). In the embodiment, the sensing electrodes 142(0), 142(1), 142(2), and 142(3) may be similar to the sensor region 142 of the physical condition sensor 140 in the foregoing embodiment, and may be respectively connected to the sensing devices 100(0), 100(1), 100(2), and 100(3) through a wired or wireless method. Each of the sensing electrodes 142(0), 142(1), 142(2), and 142(3) may be configured to detect the body temperature of the user and to generate sensing data corresponding to the detected body temperature. The sensing devices 100(0), 100(1), 100(2), and 100(3) may respectively receive the sensing data from the sensing electrodes 142(0), 142(1), 142(2), and 142(3), and transmit the sensing data to the reading device 12 via a connection 133. For example, each of the sensing devices 100(0), 100(1), 100(2), and 100(3) may include a communication interface used for performing a wired or wireless communication with the reading device 12 via the connection 133. It should be noted that there are four sensing devices 100(0), 100(1), 100(2), and 100(3) and four sensing electrodes 142(0), 142(1), 142(2), and 142(3) illustrated in FIG. 11, but the numbers of the sensing devices and the sensing electrodes may be increased or decreased according to actual requirements.

In some embodiments, as shown in FIG. 11, the number of the sensing devices 100(0), 100(1), 100(2), and 100(3), and the number of the sensing electrodes 142(0), 142(1), 142(2), and 142(3) are the same. Moreover, the reading device 12 may be a smartphone, a tablet computer, a notebook computer, a desktop computer, or other computing devices providing data transmission, data processing, and data display functions. For example, the reading device 12 may include a processor (for example, a CPU), a communication interface (for example, a wired or wireless communication circuit), a memory (for example, SRAM, DRAM, ROM, HDD, and/or SSD), a display, etc. The reading device 12 may receive the sensing data from the sensing assembly 10c and display a message related to the sensing data.

In some embodiments, a monitoring device 14 may be a smartphone, a tablet computer, a notebook computer, a desktop computer, or other computing devices providing data transmission, data processing, and data display functions. For example, the monitoring device 14 may include a processor (for example, a CPU), a communication interface (for example, a wired or wireless communication circuit), a memory (for example, SRAM, DRAM, ROM, HDD, and/or SSD), a display, etc. The monitoring device 14 may receive the sensing data from the reading device 12 and display a message related to the sensing data. The user may monitor the body temperature detected by the sensing assembly 10c by using the reading device 12 or the monitoring device 14. In other embodiments, a body temperature sensing system may not include connections 131-132 and/or the monitoring device 14.

In some embodiments, the reading device 12 may be referred to as a repeater or a transponder, such as a gateway, an access point, a modem, a base station, or a server. The reading device 12 may transmit the sensing data to the monitoring device 14 via a network (for example, the internet). For example, the reading device 12 may decode the sensing data received, then generates corresponding packets and send the packets to the monitoring device 14. The network may include public networks, private network, and/or local network.

In some embodiments, the reading device 12 may be connected to the network via a connection 131 and the monitoring device 14 may be connected to the network via a connection 132. In some embodiments, the reading device 12 may be connected to the monitoring device 14 through a peer-to-peer (P2P) connection. It should be noted that the total number of the reading device 12 and the monitoring device 14 may respectively be one or more.

In some embodiments, at least one of the connections 131, 132, and 132 may be compatible with one or more wireless communication protocols, such as Wi-Fi, Bluetooth, 3G, 4G, 5G, ZigBee, etc. In some embodiments, at least one of the connections 131, 132, and 132 may be compatible with wired communication protocol, such as the Ethernet.

In some embodiments, the sensing devices 100(0), 100(1), 100(2), and 100(3) may be paired with the reading device 12 using Wi-Fi, Bluetooth, or other wireless communication protocols. After pairing, the sensing devices 100(0), 100(1), 100(2), and 100(3), and the reading device 12 can identify each other and can establish the connection 133. In some embodiments, the reading device 12 and the monitoring device 14 may be paired with each other to perform communication.

With such configuration, when the sensing electrodes 142(0), 142(1), 142(2), and 142(3) are fixed to a plurality of body parts of the user using the binding component (for example, the binding component 400 shown in FIG. 1A), each of the sensing electrodes 142(0), 142(1), 142(2), and 142(3) may sense the body temperature of the user at the corresponding body part. The sensing data (or the temperature data) is transmitted to the reading device 12 via the sensing devices 100(0), 100(1), 100(2), and 100(3). In the embodiment, the four sensing electrodes 142(0), 142(1), 142(2), and 142(3) may be fixed to four independent and different body parts of the user, for example four different locations at the groin or foot of the user, through the binding component.

In summary of the above, the sensing assembly according to embodiments of the disclosure electrically connects to the battery module using the circuit board module covering the top surface, the bottom surface, and the side surface of the battery module. With such configuration, the circuit board module may be electrically connected to the opposite surfaces of the battery module, so that the components of the sensing device may be disposed on the opposite surfaces of the battery module, thereby improving the space utilization of the sensing device and further reducing the overall size of the sensing device.

Moreover, the printed antenna and the physical condition sensor of the sensing device may be respectively disposed on the opposite surfaces of the circuit board module to maintain a certain distance between the printed antenna and the physical condition sensor, so as to prevent interference between the two. Therefore, the sensing assembly according to embodiments of the disclosure can improve the performance of the sensing device without increasing the overall length thereof. In addition, the circuit board module may be a FPC board and thus may be bent to cover the top surface, the bottom surface, and the side surface of the battery module. Thus, the FPC board can provide flexibility to the sensing assembly, so as to provide greater comfort to the user when the sensing assembly is in direct contact with the body of the user.

In addition, through the printed antenna printed on the circuit board module, the physical condition signal sensed by the physical condition sensor may be uploaded to the cloud through a suitable wireless communication method such as Bluetooth, Wi-Fi, 4G, 5G, Long Term Evolution

(LTE), etc. In embodiments of the disclosure, the sensing assembly may use the internet (online) and the telecommunication network (offline) to perform real-time alerts and/or physical condition predictions (for example, for labor) to notify the caregiver in time.

### [Description of the Reference Signs]

10, 10a-10c: sensing assembly
12: reading device
14: monitoring device
100, 100a-100d, 100(0)-100(3): sensing device
110: battery module
112: top surface
114: bottom surface
116: side surface
120, 120a-120d: circuit board module
122: first outer surface
124: second outer surface
126: flexible printed circuit (FPC) board
127: first circuit board
127a: first pin
128: second circuit board
128a: second pin
129: connector
130: printed antenna
131-133: connection
140: physical condition sensor
142: sensor region
142(0)-142(3): sensing electrode
160: wireless communication switch
170: isolation layer
180: isolation coating
190: power source switch
192: power source pad
194: charging pad
300: inner pocket
400, 400a, 400b: binding component
400a: close-fitting bottom
410: peripheral region

## Claims

1. A sensing assembly (10, 10a-10c), comprising:
a sensing device (100, 100a-100d, 100(0)-100(3)) which comprises a battery module (110), a circuit board module (120, 120a-120d), and a physical condition sensor (140), wherein the circuit board module (120, 120a-120d) covers a top surface (112), a bottom surface (114) opposite to the top surface (112), and a side surface (116) connecting the top surface (112) and the bottom surface (114) of the battery module (110), and the physical condition sensor (140) is disposed on an outer surface of the circuit board module (120, 120a-120d) and is configured to contact a sensing region of a user to detect a physical condition signal of the sensing region; and
a binding component (400, 400a, 400b) coupled to the sensing device (100, 100a-100d, 100(0)-100(3)) to fix the sensing device (100, 100a-100d, 100(0)-100(3)) to the sensing region of the user.

2. The sensing assembly (10, 10a-10c) of claim 1, wherein the circuit board module (120) is a flexible printed circuit (FPC) board (126) bent to cover the top surface (112), the bottom surface (114), and the side surface (116).

3. The sensing assembly (10, 10a-10c) according to claim 1, wherein the circuit board module (120a) comprises a first circuit board (127), a second circuit board (128), and a connector (129), the first circuit board (127) covers the top surface (112) and comprises a plurality of first pins (127a), the second circuit board (128) covers the bottom surface (114) and comprises a plurality of second pins (128a), and the connector (129) covers the side surface (116) and opposite ends of the connector (129) are respectively engaged with the plurality of first pins (127a) and the plurality of second pins (128a) to electrically connect the first circuit board (127) and the second circuit board (128).

4. The sensing assembly (10, 10a-10c) according to claim 1, wherein the circuit board module (120b) comprises a first circuit board (127) and a second circuit board (128), the first circuit board (127) covers the top surface (112) and comprises a plurality of pins (127a), the second circuit board (128) covers the bottom surface (114) and comprises a connector (129) corresponding to the plurality of pins (127a), and the connector (129) covers the side surface (116) and engages with the plurality of pins (127a) to electrically connect the first circuit board (127) and the second circuit board (128).

5. The sensing assembly (10, 10a-10c) according to claim 1, wherein the circuit board module (120c) comprises a first circuit board (127), a FPC board (126), and a second circuit board (128), the first circuit board (127) covers the top surface (112) and is connected to the FPC board (126), the second circuit board (128) covers the bottom surface (114) and comprises a connector (129), and the flexible circuit board is bent to cover the side surface (116) and to engage with the connector (129), so as to electrically connect the first circuit board (127) and the second circuit board (128).

6. The sensing assembly (10, 10a-10c) according to claim 1, wherein the circuit board module (120d) includes an FPC board (126) and a printed circuit board (127), the FPC board (126) is bent to cover the top surface (112) and the side surface (116), the printed circuit board (127) covers the bottom surface (114) and comprises a connector (129) engaged with the FPC board (126) to electrically connect the FPC board (126) and the printed circuit board (127).

7. The sensing assembly (10, 10a-10c) according to any one of claims 1 to 6, further comprising a printed antenna (130) printed on the circuit board module (120, 120a-120d).

8. The sensing assembly (10, 10a-10c) according to claim 7, further comprising a wireless communication switch (160) disposed on the circuit board module (120, 120a-120d) and electrically connected to the printed antenna (130).

9. The sensing assembly (10, 10a-10c) according to any one of claims 1 to 8, further comprising an isolation layer (170) covering the battery module (110) and the circuit board module (120, 120a-120d), and exposing at least a portion of the physical condition sensor (140).

10. The sensing assembly (10, 10a-10c) according to claim 9, wherein a material of the isolation layer (170) comprises a thermoplastic material and the isolation layer (170) is formed by injection molding.

11. The sensing assembly (10, 10a-10c) according to any one of claims 1 to 10, wherein the sensing region is a groin of the user.

12. The sensing assembly (10, 10a-10c) according to any one of claims 1 to 11, wherein the binding component (400, 400a, 400b) comprises a close-fitting bottom (400a) and has an inner pocket (300) for accommodating the sensing device (100, 100a-100d, 100(0)-100(3)).

13. The sensing assembly (10, 10a-10c) according to any one of claims 1 to 11, wherein the binding component (400, 400a, 400b) comprises an elastic band (400b) to be fastened to a groin of the user.

14. The sensing assembly (10, 10a-10c) according to any one of claims 1 to 11, wherein the binding component (400, 400a, 400b) comprises an adhesive layer (400) disposed on a portion of the circuit board module (120, 120a-120d) covering the bottom surface (114).

15. The sensing assembly (10, 10a-10c) according to any one of claims 1 to 11, wherein the binding component (400, 400a, 400b) comprises an adhesive layer (400) disposed on a portion of the circuit board module (120, 120a-120d) covering the top surface (112) and a peripheral region (410) of the adhesive layer (400) is attached to the user.

16. The sensor device assembly (10, 10a-10c) according to any one of claims 1 to 15, wherein the sensing device (100) comprises a plurality of sensing devices (100(0)-100(3)), and the plurality of the sensing devices (100(0)-100(3)) is configured to wirelessly communicate with a reading device (12).

17. The sensing assembly (10, 10a-10c) according to any one of claims 1 to 10, wherein the sensing region is under an armpit of the user.

18. The sensing assembly (10, 10a-10c) according to any one of claims 1 to 10 and 17, wherein the binding component (400, 400a, 400b) comprises a double-sided tape (400) having one side disposed on the circuit board module (120, 120a-120d) and the other side being attached to the user.
